# EUROPEAN PATENT SPECIFICATION

(11) **EP 1 141 369 B1**
(45) Date of publication and mention of the grant of the patent: **09.04.2003**
(21) Application number: 99962574.2
(22) Date of filing: 17.12.1999
(51) Int. Cl.: C12P 13/00, C12P 41/00, C12N 9/80

(54) **PROCESS FOR THE PREPARATION OF OPTICALLY ACTIVE ALPHA-AMINONITRILES**
VERFAHREN ZUR HERSTELLUNG VON OPTISCH AKTIVEN ALPHA-AMINONITRILEN
PROCEDE DE PREPARATION D'ALPHA-AMINONITRILES ACTIVES SUR LE PLAN OPTIQUE

(30) Priority: 22.12.1998 EP 98204370
(43) Date of publication of application: 10.10.2001
(73) Proprietor: DSM N.V., 6411 TE Heerlen (NL)
(72) Inventor: QUAEDFLIEG, Peter, Jan, Leonard, Mario, NL-6164 HB Geleen (NL); SONKE, Theodorus, NL-6143 BK Sittard (NL); WAGNER, Adolf, Fritz, Volker, D-71638 Ludwigsburg (DE); BROXTERMAN, Quirinus, Bernardus, NL-6151 JA Sittard (NL); BOESTEN, Wilhelmus, Hubertus, Joseph, NL-6132 BJ Sittard (NL)
(74) Representative: Jacobs, Monique S.N.
(86) International application number: NL9900782
(87) International publication number: WO00037668

(56) References cited:
- EP-A- 0 416 282
- CHEMICAL ABSTRACTS, vol. 108, no. 13, 28 March 1988 (1988-03-28) Columbus, Ohio, US; abstract no. 108672, KANG, SHINWON ET AL: "Resolution of amino acids. XVII. Effective acyl groups on the hydrolysi of acylamino acids by mold and hog kidney acylases" XP002104184 & MEM. FAC. SCI., KYUSHU UNIV., SER. C (1987), 16(1), 61-8 CODEN: MFKCAL;ISSN: 0085-2635,
- BECKER A ET AL: "Structure of peptide deformylase and identifiation of the substrate binding site" JOURNAL OF BIOLOGICAL CHEMISTRY, vol. 273, no. 19, 8 May 1998 (1998-05-08), pages 11413-11416, XP002104183 cited in the application

## Description

The invention relates to a process for the preparation of an α-aminonitrile with enhanced optical purity wherein a mixture of the enantiomers of the N-formyl-α-aminonitrile is brought into contact with an acylase, whereby one of the enantiomers of the N-formyl α-aminonitrile is selectively deformylated into the unprotected corresponding α-aminonitrile.

There are no processes known in the art wherein a mixture of the enantiomers of an α-aminonitrile is enzymatically, enantioselectively formylated, or wherein a mixture of the enantiomers of an N-formyl-α-aminonitrile is deformylated.

Applicant now has found that it is possible to remove the N-protecting formyl group enantioselectively from one of the enantiomers of a mixture of the enantiomers of N-formyl-α-aminonitriles. In such enantioselective processes moreover very high E-values can be obtained.

α-Aminonitriles to be used as a substrate in the process of the invention are for instance aliphatic and aromatic α-aminonitriles, for example the α-aminonitriles derived from phenylglycine, phenylalanine, *m*-methoxy-phenylalanine, valine and α-methyl-phenylglycine. In the framework of this invention an α-aminonitrile is understood to be an α-aminoacid wherein the carboxy group is replaced by a cyano group.

N-formyl-α-aminonitriles to be used as a substrate are for instance nitriles of formula 1 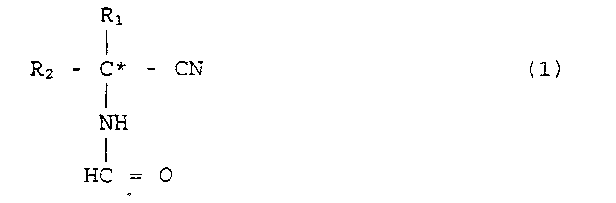 wherein:
R₁ represent an, optionally substituted, alkyl or aryl group
R₂ represents H, an, optionally substituted, alkyl or aryl group.

The alkyl groups in R₁ and R₂ may be cyclic or linear or branched chains. The alkyl and aryl groups may be substituted. Suitable substituents are for instance, hydroxy, alkyl, alkoxy, e.g. methoxy, mercapto, alkylmercapto, amino, guanyl, carboxamide, halogen, e.g. chloro, aryl e.g., phenyl and hydroxy phenyl, imidazolyl or indolyl.

In another embodiment of the present invention a mixture of the enantiomers of a (non-protected) α-aminonitrile is subjected to a formylation in the presence of an acylase and a formylating agent, whereby one of the enantiomers is selectively converted into the corresponding N-formyl α-aminonitrile.

Suitable formylating agents are for instance formic acid in case a thermodynamically controlled formylation can be performed, or formic acid esters or amides when the formylation is kinetically controlled. In a thermodynamically controlled formylation the equilibrium is shifted towards the side of the formyl derivative, preferably by precipitation of the formyl derivative.

Moreover it appeared that, starting from α-aminonitriles, the non-formylated α-aminonitriles relatively rapidly racemise at pH values of higher than 5. In such case the optically active N-formyl aminonitrile can be obtained with an enantiomeric excess of more than 90%, in particular more than 95% and with a yield of more than 90%, in particular more than 95%, calculated with respect to the total amount of (racemic) α-aminonitrile starting product.

Suitable acylases that can be used in the process of the present invention are for instance Penicilline acylases for instance Pen-G or Pen-V acylases, metalloproteases, esterases, deacetylases. Particularly useful enzymes are peptide deformylases.

Peptide deformylases (PDF's) are in general enzymes having formyl methionine peptide deformylase activity. The peptide deformylases to be used according to the invention have a more than 10 times, preferably more than 100 times, in particular more than 1000 times, higher activity towards the N-formyl protected α-aminonitriles compared to the corresponding N-acetyl protected α-amino nitriles. Activity here is defined as the catalytic efficiency (also called: specificity constant) K_{cat}/Kₘ expressed in M⁻¹ sec⁻¹; wherein Kₘ (expressed in mM) represents the Michaelis constant (this is the substrate concentration at which the reaction rate is 50% of the maximum reaction race observed) and K_{cat} (expressed in min⁻¹) represents the turnover number. It should be noticed that in the literature also other names are being used instead of the name peptide deformylases; in particular the following names may be mentioned here: formylmethionine deformylase, N-formylmethionylaminoacyl-tRNA deformylase, N-formyl-L-methionine amidohydrolase, N-formylmethionyl-aminoacyl-tRNA amidohydrolase.

Suitable peptide deformylases to be used in the process according to the invention are peptide deformylases classified as EC 3.5.1.27. Preferably, the enzyme is an enzyme having the activity as described for EC 3.5.1.27 because excellent results are being achieved in the deformylation with such enzymes. It should be noticed that until recently it was believed that the enzyme coded as EC 3.5.1.31 is catalyzing a different reaction. In the meantime, however, it has been shown that the enzymes known as EC 3.5.1.27 and EC 3.5.1.31 are coded for by exactly the same gene and have the same activity. Therefore, as used herein, the term EC 3.5.1.27 is encompassing not only EC 3.5.1.31, but likewise all other enzymes having the same activity as described for EC 3.5.1.27.

Although the family of PDF's is composed of proteins with a relatively low level of sequence identity, the 3D structures of the members of this family appear closely related one to each other with, in particular, the building of a common fold around the bivalent metal ion and three signature sequences. As is described (for PDF's indicated as PDF) by Wagner et al., J. Biol. Chem., 273, 11413-6 (1998), for many of these enzymes characteristically three short amino acid stretches are present as strictly conserved motifs, namely in that the enzymes contain the sequences (i) HEXXH, (ii) EGCLS and (iii) GXGXAAXQ. In these sequences X represents any natural amino acid, and standard one letter codes for amino acids are used: A = alanine, C = cysteine, E = glutamic acid, G = glycine, H = histidine, L = leucine, S = serine and Q = glutamine.

Peptide deformylases are obtainable for instance from eubacteria for example *Escherlchia coli, Bacillus subtills, Clostridium acetobutylicum, Clostridium beijerinckii, Haemophilus influenzae, Thermotoga maritima, Thermus aquaticus, Thermus thermophilus, Calothrix PCC 7601, Bacillus stearothermophilus, or Lactococcus lactis.* Preferably an enzym obtainable from *Escherichia coli* is used.

Preferably a peptide deformylase is used with a bivalent metal ion whereby the metal is chosen from the groups 5-11 of the periodic system (New IUPAC version; see Handbook of Chemistry and Physics 70th edition, CRC Press, 1989-1990, inner page of cover), as a cofactor. Preferably the metal is chosen from the group of V, Cr, Fe, Ni, Mn, Co, Cu, Pd and Pt, in particular from the group of Fe, Ni, Mn and Co, most preferably Fe or Ni.

Preferably the amount of the bivalent metal ions should be about equivalent to the number of moles of enzyme. Suitably the molar ratio between these bivalent metal ions and the number of PDF molecules is in the range of 0.6 to 1.4, preferably of 0.8 to 1.2, and most preferred the amount of bivalent metal ions is equimolar to the enzyme.

Exchange of the bivalent metal ions in the PDF's in order to obtain PDF enzymes with a co-factor as necessary for the present invention can be done by the various methods as described in Groche et *al.,* Biochem. Biophys. Res. Comm., 246, 342-346, (1998). These methods include simple metal displacement by incubation of the native enzyme in an excess of the desired bivalent metal ion, if necessary preceeded by the preparation of the apoenzyme via treatment of the native enzyme with a metal chelation compound. Furthermore, the desired bivalent metal ion can already be introduced in (at least part of the enzyme molecules) by using a bacterial growth medium with an enhanced ratio of the desired bivalent metal ion over Fe²⁺.

In addition measures may be taken in order to enhance the stability of the enzyme, for instance the addition of stabilisation agents, for instance catalase, tris-(2-carboxyethyl)phosphine, glucose oxidase, or combinations thereof; or enlarging the concentration of the PDF, for instance to a PDF concentration of at least 0.1 mg of PDF per ml, more preferably of at least 1.0 mg/ml. The upper limit of the concentration of PDF is not critical if practical concentrations are being used. The use of stabilisation measures is especially preferred when an easily oxidisable metal ion, e.g. Fe⁺⁺ is present as a cofactor or an easily oxidisable substrate. If not, for instance in case Ni⁺⁺ is present as a cofactor, the addition of a stabilisation agent appeared to be superfluous, as the enzyme turned out to be very stable even without stabilisation agent.

In addition measures may be taken in order to enhance the stability of the enzyme, for instance the addition of stabilisation agents, for instance catalase, tris-(2-carboxyethyl)phosphine, glucose oxidase, or combinations thereof; or enlarging the concentration of the PDF, for instance to a PDF concentration of at least 0.1 mg of PDF per ml, more preferably of least 1.0 mg/ml. The upper limit of the concentration of PDF is not critical if practical concentrations are being used. The use of stabilisation measures is especially preferred when an easily oxidisable metal ion, e.g. Fe⁺⁺ is present as a cofactor or an easily oxidisable substrate. If not, for instance in case Ni⁺⁺ is present as a cofactor the addition of a stabilisation agent appeared to be superfluous, as the enzyme turned out to be very stable even without stabilisation agent.

Alternatively, genetically engineered mutants of PDF's may be used which have for instance enhanced activity or enantioselectivity in the (de)formylation reaction. These mutants can be generated by a number of different approaches; for instance, by site-directed mutagenesis, site-specific random mutagenesis, regio-specific random mutagenesis, and completely random mutagenesis; the latter form of mutagenesis is better known as directed evolution. General applicable methods to perform these different protein engineering approaches are well known to the skilled man. If a random approach will be applied, the mutagenesis cycle will need to be followed by selection of resistent and active mutant(s), thereby leading to the identification of suitable mutants. To obtain PDF mutants also a combination of different protein engineering approaches and/or several rounds of random mutagenesis may be used.

The reaction conditions for the enzymatic deformylation or formylation according to the invention are not very critical and may depend on the substrates used. Any suitable solvent system which is inert towards the PDF may be applied; such solvents include aqueous systems (solutions or slurries) or aqueous systems also containing a water-miscible organic solvent which is inert under the reaction conditions. Aqueous systems, however, are preferred. Also the concentration of the N-formyl compound is not critical, and may be for instance in the range of about 0.1 to 1000 mM. It is not necessary that all of the N-formyl compound is dissolved; part of it may be present as a slurry. The concentration of the PDF likewise is not very critical, and usually will be at 0.001 to 100 % by weight of the formyl compound, e.g. at about 0.2 mM of PDF. The pH for the reaction preferably is chosen in the range of 4.0 to 11.0, more preferably of 5.0 to 10.0. The optimum pH is determined by the stability of the α-aminonitrile and/or the N-formyl-α-aminonitrile, and/or the stability and/or activity of the enzyme. The person skilled in the art can easily determine the optimum pH-value. The temperature is not very critical, and suitably will be in the range of 10 to 50°C, e.g. at about 37°C, but for thermostable PDF enzymes higher temperatures may be applied.

In those cases wherein the absolute configuration of the (de)formylated enantiomer was determined, it appeared that the S-enantiomer was (de)formylated more rapidly than the R-enantiomer. The optical purity is given by the enantiomeric excess (ee), the enantioselectivity of the enzyme is represented by E, and calculated as k_{f}/kₛ wherein k_{f} is defined as the rate constant of (de)formylation of the most rapidly (de)formylated enantiomer and kₛ is defined as the rate constant of (de)formylation of the least rapidly (de)formylated enantiomer.

Optionally a salt promoting hydrophobic interactions is added to the reaction mixture, for instance a sulphate, phosphate, sulphite or acetate of ammonium, Rb, K, Na, Cs or Li. Most preferably ammonium sulphate or lithium sulphate is used.

The invention will further be elucidated by the following 3 examples, without being limited thereto.

### Abbreviations:

| | |
|---|---|
| TB medium | 12 g/l of Bacto-Tryptone, Difco; 24 g/l of yeast extract, Difco; 4 g/l of glycerole; 2.3 g/l of KH₂PO₄; 12.5 g/l of K₂HPO₄); |
| Hepes | *N*-2-hydroxyethylpiperazine-*N*'-2-ethane sulphuric acid; |
| AEBSF | 2-aminoethyl-p-benzene sulphonyl fluoride; |
| TCEP | tris-(2-carboxyethyl)-phosphine. |
| MOPS | 3-(N-morpholino) propane sulphonic acid |
| MES | 2-(N-morpholino) ethane sulphonic acid |

### Isolation of PDF(Fe)

For a detailed discussion of the methods used reference is made to Groche et al., BBRC 246, 342-346 (1998).

PDF(Fe) was isolated from overproducing *E.coli* cells grown at 30°C in 1.6 1 TB medium for 14-16 h. About 13 g (wet weight) cell paste were suspended in 26 ml buffer (20 mM Hepes/KOH, 100 mM KF, pH 7.7 supplemented with 10 µg/ml catalase from bovine liver (Boehringer Mannheim) and 1 mM AEBSF, disintegrated by sonication (Branson B12, 20 min) at 0°C and centrifuged at 200.000g for 1 h. The clear supernatant (1.3 g of protein; according to biurete reaction) was mixed with 1.3 ml 10%(w/v) Polymin G-35 (BASF) adjusted to pH 7.7 and centrifuged at 40.000g for 10 min. The supernatant was applied to a 20 ml Met-Lys-Sepharose column that had been equilibrated with 20 mM Hepes/KOH, 100 mM KF, 0.2 mM TCEP, pH 7.7. After washing with 120 ml of 20 mM Hepes/KOH, 100 mM KF, 0.2 mM TCEP, pH 7.7, PDF(Fe) was eluted with 150 ml 20 mM Hepes/KOH, 100 mM KCl, 0.2 mM TCEP, pH 7.7. The protein containing fractions were concentrated by ultrafiltration using an Amicon PM10 membrane (yield: 140 mg protein, 1400 U/mg; determined according to Groche et al). After adjustment of the TCEP concentration to 1 mM and protein concentration to 40 mg/ml, the PDF(Fe) stock solution (40 mg/ml = 2 mM) was stored frozen at -60°C.
After thawing, the PDF(Fe) stock solution could be used directly in the deformylation experiments described below. If however solutions with lower PDF(Fe) concentrations were required for these deformylation experiments, the PDF stock solution was diluted further in 20 mM Hepes/KOH, pH 7.7, 100 mM KCl, 1 mg/ml bovine serum albumin, 10 µg/ml catalase solution.

### HPLC-analysis

In all cases HPLC conditions had to be developed in which the two deformylated isomers were separated from each other and from the formylated isomers. To this end two different techniques were applied, i.e. method A and method B, as described below.

From the concentrations of deformylated isomers in the samples after various reaction times, the (de) formylation rate constant (k_{f} and kₛ in M⁻¹s⁻¹) could be calculated for both enantiomers, as well as the respective ee values of the deformylated product. The enantioselectivity of the enzyme (E value) was calculated by taking the ratio k_{f}/kₛ and is given, as well as the maximum ee value of the deformylated product observed during the experiments, in the examples below.

### Method A (without derivatization)

A Crownpak™ CR(+) column (4x150 mm) was used. Samples (5 µl) withdrawn from the deformylation mixture were mixed with 95 µl aqueous HClO₄ (10 mM) to inactivate PDF(Fe²⁺). Following a brief centrifugation, 20 µl of the supernatant were applied to the Crownpak™ CR(+) column. For specific chromatographic conditions and retention times see the examples II and III.

### Method B (Precolumn derivatization with o-Phthaldialdehyde (OPA) and N-acetyl-L-cysteine: (NAC))

Samples (25 µl) withdrawn from the deformylation mixture were mixed with 25 µl aqueous HClO₄ (100 mM) to inactivate PDF (Fe²⁺). Following a brief centrifugation, 40 µl of the supernatant were added to 80 µl 1 M aqueous H₃BO₃/NaOH pH 11, and subsequently 20 µl OPA reagent (consisting of OPA in H₂O/CH₃OH 1:1 v/v with a concentration as indicated in the example) was added, and 10 minutes later 20 µl NAC reagent (consisting of NAC in H₂O/CH₃OH 1:1 v/v with a concentration as indicated in the example) was added. After 5 min derivatization was terminated by addition of 80 µl (500 mM) aqueous H₃PO₄, and 20 µl of the solution were instantaneously applied to a Nucleosil™ 120-5 C₁₈ column (250 x 4 mm). Temperature is ambient and detection is spectrophotometric using a wavelength of 257 nm and/or 340 nm; the used eluent is a mixture of 80 mol% aqueous 0.05 M H₃PO₄ (brought at pH = 7.0 with 1 M NaOH) and 20 vol% CH₃CN.

### Example I: Deformylation of N-formyl-valine aminonitrile in the presence of Li₂SO₄ at pH = 7.2.

The deformylation reaction of *N*-formyl-valine aminonitrile was performed in a 1.5 ml Eppendorf reaction test tube. The reaction mixture with a total volume of 200 µl contained 100 mM aqueous MOPS/NaOH, 2 M Li₂SO₄ buffer pH 7.2, and 10 mM of N-formyl-valine aminonitrile. After thermal equilibration to 37°C the deformylation reaction was started by the addition of 50 µM of PDF. At various reaction times samples of the reaction mixture were withdrawn in which the reaction was stopped by addition of HClO₄.
HPLC-analysis was performed according to method B, with [OPA] = 16 mg/ml, and [NAC] = 4 mg/ml, retention times: 8.6 min (L-enantiomeer), 10.2 min (D-enantiomeer).

### Results:

E = 47.9
eeₘₐₓ = 95.5
kₛ = 0.62 M⁻¹s⁻¹
k_{f} = 29.7 M⁻¹s⁻¹

### Example II: Deformylation of N-formyl-m-methoxyphenylalanine aminonitrile without Li₂SO₄ at pH 7.2

The deformylation reaction of *N*-formyl-*m*-methoxy-phenylalanine aminonitrile was performed as described in example I, with the exception that 100 mM MOPS/NaOH, 250 mM NaCl, 0.1 mg/ml catalase buffer pH 7.2 was used instead of 100 mM MOPS/NaOH, 2 M Li₂SO₄ buffer pH 7.2. Futhermore, 7.2 mM of N-formyl-m-methoxy-phenylalanine aminonitrile and 2.5 µM of PDF were used.
HPLC-analysis was performed according to method A Eluent: 90 vol% 10 mM aqueous HClO₄/10 vol% CH₃OH Flow rate: 0.8 ml/min, temperature: 5° C, detection: 210 nm,
retention times:

| | |
|---|---|
| Deformylated enantiomer(s) | 23.8 min. |
| | 30.7 min. |
| N-formyl aminonitrile | 52.0 min. |

### Results:

E = 685
eeₘₐₓ = 99.0
k_{f} = 1370 M⁻¹s⁻¹
kₛ = 2 M⁻¹s⁻¹

### Example III: Deformylation of N-formyl-phenylalanine aminonitrile without Li₂SO₄ addition at pH 6.2.

The deformylation reaction of *N*-formyl-phenylalanine aminonitrile was performed as described in example I, with the exception that 100 mM MES/NaOH buffer pH 6.2 was used instead of 100 mM MOPS/NaOH, 2 M Li₂SO₄ buffer pH 7.2. Furthermore, 7.5 mM of *N*-formyl-phenylalanine aminonitrile and 20 µM of PDF were used.
HPLC-analysis was performed according to method A Eluent: 90 vol% 10 mM aqueous HClO₄/10 vol% CH₃OH Flow rate: 0.8 ml/min, temperature: 5° C, detection: 210 nm,
retention time:

| | |
|---|---|
| deformylated aminonitrile | 11.8 min |
| | 15.1 min |
| N-formyl aminonitrile | 28.6 min. |

### Results:

E = 880
eeₘₐₓ = 98.8
k_{f} = 880
kₛ = 1

## Claims

1. Process for the preparation of an α-aminonitrile with enhanced optical purity wherein a mixture of the enantiomers of a chiral N-formyl α-aminonitrile is brought into contact with an acylase, whereby one of the enantiomers of the N-formylaminonitrile is selectively deformylated into the unprotected corresponding α-aminonitrile.

2. Process for the preparation of an α-aminonitrile with enhanced optical purity wherein a mixture of the enantiomers of a chiral (unprotected) α-aminonitrile is subjected to a formylation reaction in the presence of an acylase and a formylating agent whereby one of the enantiomers is selectively converted in N-formyl α-aminonitrile.

3. Process according to claim 2 wherein formic acid, a formic acid amide or a formic acid ester is used as a formylating agent.

4. Process according to any of claims 1-3, wherein a peptide deformylase with a bivalent metal ion wherein the metal is chosen from group 5-11 of the periodic system, is used as acylase.

5. Process according to any of claims 1-4, wherein the peptide deformylase is chosen from the class EC 3.5.2.27 or EC 3.5.1.31.

6. Process according to any of claims 1-5, wherein the peptide deformylase contains the sequences of (I) HEXXH, (ii) EGCLS and (iii) GXGXAAXQ.

7. Process according to any of claims 4-6, wherein the peptide deformylase is from *Escherichia coli.*

8. Process according to any of claims 4-7, wherein the bivalent metal is chosen from the group of Fe, Ni, Mn and Co.

9. Process according to claim 8, wherein the bivalent metal is Ni.

10. Process according to any of claims 1-8, wherein in addition a stabilisation agent is added.

11. Process according to claim 10 wherein the stabilisation agent is catalase.

12. Process according to claim 10 or 11 wherein the bivalent metal is Fe.

## Patentansprüche

1. Verfahren für die Herstellung eines α-Aminonitrils mit erhöhter optischer Reinheit, wobei ein Gemisch aus den Enantiomeren eines chiralen N-Formyl-α-aminonitrils mit einer Acylase in Kontakt gebracht wird, wodurch eines der Enantiomere des N-Formylaminonitrils selektiv in das ungeschützte entsprechende α-Aminonitril deformyliert wird.

2. Verfahren für die Herstellung eines α-Aminonitrils mit erhöhter optischer Reinheit, wobei ein Gemisch aus den Enantiomeren eines chiralen (ungeschützten) α-Aminonitrils in Gegenwart einer Acylase und eines Formylierungsmittels einer Formylierungsumsetzung unterworfen wird, wodurch eines der Enantiomere selektiv in N-Formyl-α-aminonitril umgewandelt wird.

3. Verfahren gemäß Anspruch 2, wobei Ameisensäure, ein Ameisensäureamid oder ein Ameisensäureester als Formylierungsmittel verwendet wird.

4. Verfahren gemäß einem der Ansprüche 1-3, wobei eine Peptiddeformylase mit einem bivalenten Metall-Ion, worin das Metall aus Gruppe 5-11 des Periodensystems ausgewählt wird, als Acylase verwendet wird.

5. Verfahren gemäß einem der Ansprüche 1-4, wobei die Peptiddeformylase aus der Klasse EC 3.5.2.27 oder EC 3.5.1.31 ausgewählt wird.

6. Verfahren gemäß einem der Ansprüche 1-5, wobei die Peptiddeformylase die Sequenzen (I) HEXXH, (ii) EGCLS und (iii) GXGXAAXQ enthält.

7. Verfahren gemäß einem der Ansprüche 4-6, wobei die Peptiddeformylase von *Escherichia coli* stammt.

8. Verfahren gemäß einem der Ansprüche 4-7, wobei das bivalente Metall ausgewählt wird aus der Gruppe von Fe, Ni, Mn und Co.

9. Verfahren gemäß Anspruch 8, wobei das bivalente Metall Ni ist.

10. Verfahren gemäß einem der Ansprüche 1-8, wobei zusätzlich ein Stabilisierungsmittel zugegeben wird.

11. Verfahren gemäß Anspruch 10, wobei das Stabilisierungsmittel Katalase ist.

12. Verfahren gemäß Anspruch 10 oder 11, wobei das bivalente Metall Fe ist.

## Revendications

1. Procédé pour la préparation d'un α-aminonitrile avec une pureté optique améliorée dans lequel on porte un mélange des énantiomères d'un N-formyl-α-aminonitrile chiral en contact avec une acylase, et ainsi un des énantiomères du N-formylaminotrile est sélectivement déformylé en l'α-aminonitrile non protégé correspondant.

2. Procédé pour la préparation d'un α-aminonitrile avec une pureté optique améliorée dans lequel on soumet un mélange des énantiomères d'un α-aminonitrile (non protégé) chiral à une réaction de formylation en présence d'une acylase et d'un agent de formylation, ce qui permet à un des énantiomères d'être sélectivement transformé en le N-formyl-α-aminonitrile.

3. Procédé selon la revendication 2, dans lequel on utilise l'acide formique, l'amide d'acide formique ou un ester d'acide formique à titre d'agent de formylation.

4. Procédé selon l'une quelconque des revendications 1 - 3, dans lequel on utilise à titre d'acylase, une peptide déformylase avec un ion de métal bivalent, le métal étant choisi dans le groupe 5 - 11 du Système Périodique.

5. Procédé selon l'une quelconque des revendications 1 - 4, dans lequel la peptide déformylase est choisie dans la catégorie EC 3.5.2.27 ou EC 3.5.1.31.

6. Procédé selon l'une quelconque des revendications 1 - 5, dans lequel la peptide déformylase contient les séquences de (i) HEXXH, (ii) EGCLS et (iii) GXGXAAXQ.

7. Procédé selon l'une quelconque des revendications 4 - 6, dans lequel la peptide déformylase provient de Escherichia coli.

8. Procédé selon l'une quelconque des revendications 4 - 7, dans lequel le métal bivalent est choisi dans le groupe comprenant Fe, Ni, Mn et Co.

9. Procédé selon la revendication 8, dans lequel le métal bivalent est Ni.

10. Procédé selon l'une quelconque des revendications 1 - 8, dans lequel on ajoute en plus un agent de stabilisation.

11. Procédé selon la revendication 10, dans lequel l'agent de stabilisation est une catalase.

12. Procédé selon la revendication 10 ou 11, dans lequel le métal bivalent est Fe.
